# FASCICULE DE BREVET EUROPEEN

(11) **EP 4 478 994 B1**
(45) Date de publication et mention de la délivrance du brevet: **21.01.2026**
(21) Numéro de dépôt: 23702973.1
(22) Date de dépôt: 27.01.2023
(51) Int. Cl.: A61F 2/44, A61F 2/46, A61F 2/30

(54) **DISPOSITIF D'IMPLANT POUR LA REALISATION D'UNE ARTHRODESE RACHIDIENNE PAR VOIE POSTERIEURE AU NIVEAU D'UNE ARTICULATION FACETTAIRE, ET SYSTEME COMPRENANT LEDIT IMPLANT ET SON OUTIL DE POSE**
IMPLANTATVORRICHTUNG ZUR DURCHFÜHRUNG EINER POSTERIOREN WIRBELSÄULENARTHRODESE AN EINEM FACETTENGELENK UND SYSTEM MIT DEM IMPLANTAT UND SEINEM POSITIONIERWERKZEUG
IMPLANT DEVICE FOR PERFORMING POSTERIOR SPINAL ARTHRODESIS AT A FACET JOINT, AND SYSTEM COMPRISING THE IMPLANT AND ITS POSITIONING TOOL

(30) Priorité: 17.02.2022 FR 2201374
(43) Date de publication de la demande: 25.12.2024
(73) Titulaire: SC Medica, 67000 Strasbourg (FR)
(72) Inventeur: SROUR, Camille, 67200 STRASBOURG (FR)
(74) Mandataire: Cabinet Bleger-Rhein-Poupon
(86) Numéro de dépôt international: PCT/EP2023/052040
(87) Numéro de publication internationale: WO 2023/156169

(56) Documents cités:
- EP-A1- 0 912 147
- WO-A1-98/48738
- US-A1- 2019 328 429
- US-B1- 6 443 990

## Description

La présente a pour objet un dispositif d'implant pour la réalisation d'une arthrodèse rachidienne par voie postérieure au niveau d'une articulation facettaire, et un système comprenant ledit dispositif d'implant et l'outil permettant sa pose.

Une arthrodèse rachidienne consiste à fusionner deux vertèbres afin de les immobiliser.

La voie postérieure est la plus aisée, on utilise soit une plaque ou une barre d'ostéosynthèse reliant les vertèbres en y étant fixée au moyen de vis pédiculaires, soit des cages intersomatiques du type de celles décrites par exemple dans les documents US 2011/230965, FR 2 946 245 et WO 02/03895, destinées à prendre place dans l'espace intervertébral après enlèvement du disque vertébral.

On peut également immobiliser deux vertèbres, en procédant également par voie postérieure, par la fusion de chacune des apophyses articulaires inférieures d'une vertèbre avec l'apophyse articulaire supérieure en regard, de la vertèbre inférieure voisine.

Cette arthrodèse rachidienne par voie postérieure au niveau d'une articulation facettaire, présente des avantages par rapport aux autres techniques fréquemment mises en œuvre, notamment en ce qu'elle est moins traumatique et présente moins de risques.

Cette technique nécessite toutefois d'utiliser des moyens différents de ceux des autres techniques opératoires, et notamment des cages présentant des caractéristiques différentes de celles des documents précités.

Une telle cage doit notamment présenter une certaine finesse du fait de l'étroitesse de l'espace dans lequel elle doit être introduite. De ce fait la principale difficulté est relative à la fixation de cette cage.

On connaît déjà des dispositifs d'implant destinés à être insérés dans une articulation facettaire, et notamment celui décrit dans le document WO 2012154653, qui consiste en un élément en forme de coin, dont les deux faces opposées, destinées chacune à venir au contact d'une facette, présentent une surface munie de reliefs pour en assurer l'ancrage, et qui est percé d'une multiplicité de trous destinés au développement de la matière osseuse après dépôt d'un greffon osseux, tandis que la fixation est réalisée au moyen de deux vis, chacune vissée dans une apophyse, obliquement par rapport au plan médian de l'implant. Ce dispositif d'implant reste complexe, ou plus particulièrement, c'est sa mise en œuvre qui est complexe.

On connaît également par le document WO2019016341 au Nom de la présente Demanderesse, un implant qui se présente sous la forme d'un élément plat, d'épaisseur constante, comprenant, pour la réception d'un greffon osseux, une large ouverture centrale lui conférant une forme sensiblement annulaire, et destiné à être introduit dans une cavité préalablement réalisée à l'emplacement de l'articulation facettaire à immobiliser, ledit élément présentant une forme de D, le côté arrondi constituant le bord distal d'introduction dans ladite cavité, tandis que le bord opposé, proximal rectiligne, comporte un moyen de solidarisation à un outil de maintien et de mise en place, tandis que les faces opposées dudit élément plat, comportent des reliefs qui consistent en des crans disposés perpendiculairement au sens d'introduction, chacun des crans présentant un profil de dent à deux pans dont l'un, celui orienté du côté dudit bord proximal est perpendiculaire ou sensiblement perpendiculaire au plan principal dudit élément, tandis que l'autre pan est incliné.

L'épaisseur constante empêche une expulsion, telle que cela peut survenir lorsque la cage est en forme de coin. Et du point de vue mise en œuvre, les travaux préparatoires de façonnage de la cavité sont réalisés de manière la plus simple possible, c'est-à-dire que dans la cavité les faces en regard sont plates et parallèles.

En pratique le chirurgien prépare l'emplacement de l'implant en enlevant la capsule articulaire à l'aide d'un instrument appelé ciseau facettaire, puis utilise une râpe pour faire "saigner" les parties en regard de l'interligne sur les facettes. Cette étape avec la râpe a deux buts : préparer l'espace préexistant, avec une interligne sans os mais souvent un peu courbée, à la forme droite de l'implant, et favoriser la fusion ultérieure des facettes à travers l'implant en ayant fait saigner/raviver les facettes jusqu'à arriver à l'os spongieux.

On connaît également par les documents EP0912147 ou WO9848738, des implants intersomatiques utilisables dans le traitement chirurgical du rachis, destinés à être disposés dans l'espace intervertébral et non au niveau d'une articulation facettaire.

Ces implants se présentent sous la forme d'une cage dans laquelle peut tourillonner une vis dont au moins une partie est d'un diamètre supérieur à l'épaisseur de ladite cage, en sorte de former des moyens d'ancrage. La mise en place d'un tel implant nécessite des travaux de préparation importants, difficilement réalisables au niveau d'une articulation facettaire.

Dans le cas de l'implant du document EP0912147, la vis est vissée dans une partie annulaire taraudée localisée en périphérie de la cage, et par conséquent de diamètre externe plus grand que l'épaisseur de la cage, ce qui nécessite un usinage particulier de la matière osseuse pour loger ladite partie annulaire.

Le document WO9848738 propose un implant similaire, dont les faces présentent une simple ou double conicité en vue d'une adaptation aux caractéristiques des corps vertébraux.

Si les moyens d'ancrage dans la matière osseuse de ces implants permettent de s'affranchir de l'utilisation de vis, introduites obliquement par exemple, aucun de ces implants n'est adapté à la réalisation d'une arthrodèse rachidienne par voie postérieure au niveau d'une articulation facettaire, laquelle présente des contraintes de mise en place, parce que l'espace dans lequel l'implant doit être introduit est de dimensions réduites, notamment en ce qui concerne l'épaisseur.

La présente invention selon la revendication 1 a pour but de remédier aux divers inconvénients précités, en proposant un dispositif d'implant, ainsi qu'un système comprenant ledit implant et son outil de pose, pour la réalisation d'une arthrodèse rachidienne par voie postérieure au niveau d'une articulation facettaire.

Le dispositif d'implant pour la réalisation d'une arthrodèse rachidienne par voie postérieure au niveau d'une articulation facettaire, selon l'invention, se présente sous la forme d'un élément plat, d'épaisseur constante, destiné à être introduit dans une cavité préalablement réalisée à l'emplacement de l'articulation facettaire à immobiliser, comprenant sur chacune de ses faces des reliefs formant des crans constituant des moyens d'ancrage, et associé à au moins un moyen d'ancrage complémentaire, constitué d'une vis d'un diamètre supérieur à l'épaisseur dudit élément, conçue apte à être vissée dans ledit élément plat en sorte qu'au moins une partie de ses filets déborde desdits faces, et il se caractérise en ce qu'il présente une forme de U, à savoir qu'il comporte une partie transversale, de laquelle s'étendent deux branches séparées par un espace, et où ladite partie transversale constitue le bord distal d'introduction dans ladite cavité, tandis que du côté proximal les extrémités libres desdites branches comportent chacune, du côté intérieur du U, des moyens de retenue aptes à permettre le vissage et l'introduction entre lesdites branches, de ladite vis, et qui font saillie de chacun des côtés internes desdites branches pour s'étendre dans ledit espace, sans déborder l'épaisseur dudit élément plat.

La forme en U du dispositif d'implant, c'est-à-dire le fait qu'il soit ouvert du côté proximal, et qu'il ne comporte pas d'élément annulaire de retenue d'une vis, permet d'obtenir un élément, outre qu'il soit de faible épaisseur, qui ne nécessite pas d'usinage spécifique.

Par ailleurs, l'axe de la vis est coaxial à celui d'introduction du dispositif d'implant, ce qui permet d'envisager un abord percutané.

La présente invention concerne également un système pour la réalisation d'une arthrodèse rachidienne par voie postérieure au niveau d'une articulation facettaire, comportant un dispositif d'implant et son outil de pose, dans lequel ledit implant se présente sous la forme d'un élément plat, d'épaisseur constante, destiné à être introduit dans une cavité préalablement réalisée à l'emplacement de l'articulation facettaire à immobiliser, comprenant sur chacune de ses faces des reliefs formant des crans constituant des moyens d'ancrage, et associé à au moins un moyen d'ancrage complémentaire, constitué d'une vis d'un diamètre supérieur à l'épaisseur dudit élément, conçue apte à être vissée dans ledit élément plat en sorte qu'au moins une partie de ses filets déborde desdits faces, et il se caractérise en ce que :
- ledit implant présente une forme de U, à savoir qu'il comporte une partie transversale, de laquelle s'étendent deux branches, et où ladite partie transversale constitue le bord distal d'introduction dans ladite cavité, tandis que du côté proximal les extrémités libres desdites branches comportent chacune, du côté intérieur du U, des moyens de retenue aptes à permettre le vissage et l'introduction entre lesdites branches, de ladite vis,
- ledit outil de pose comprend un ensemble de pièces coaxiales à savoir, d'une part une première pièce dont une extrémité est apte à tenir ledit élément plat en le maintenant par l'extérieur de ses branches, d'autre part une deuxième pièce dans laquelle est apte à coulisser ladite première pièce jusqu'à extraction de la partie de cette dernière tenant ledit élément plat ; d'autre part encore intérieurement à ladite première pièce, une troisième pièce dont une extrémité est apte à être connectée audit élément plat et à maintenir ladite vis en regard desdites branches ; et d'autre part encore, intérieurement à ladite troisième pièce, une quatrième pièce qui consiste en une tige dont une extrémité est configurée pour engager ladite vis et pouvoir l'entraîner en rotation au travers d'une action sur l'autre extrémité.

Selon une caractéristique additionnelle du système selon l'invention, l'extrémité de la première partie, apte à tenir l'élément plat en le maintenant par l'extérieur de ses branches, comporte deux bras parallèles, conçus aptes à enserrer lesdites branches par l'extérieur.

Selon une autre caractéristique additionnelle du système selon l'invention, les branches de l'élément plan présentent chacune extérieurement une gorge longitudinale de guidage pour les bras de la première pièce, et en ce que les fonds desdites gorges et l'intérieure desdits bras présentent des reliefs complémentaires aptes à immobiliser axialement ledit élément entre lesdits bras lorsque ceux-ci sont maintenus extérieurement dans la deuxième pièce.

Selon une autre caractéristique additionnelle du système selon l'invention, les reliefs consistent en des bossages.

Selon une autre caractéristique additionnelle du système selon l'invention, la troisième pièce est de forme tubulaire tandis que son extrémité apte à maintenir la vis en attente, consiste en une cavité de section transversale ronde et taraudée.

Selon une autre caractéristique additionnelle du système selon l'invention, l'outil comporte une pièce supplémentaire, utilisable dans une intervention percutanée, et intégrant toutes les autres pièces.

Les avantages et les caractéristiques du dispositif d'implant selon l'invention ainsi que du système pour la réalisation d'une arthrodèse rachidienne par voie postérieure au niveau d'une articulation facettaire selon l'invention, ressortiront plus clairement de la description qui suit et qui se rapporte au dessin annexé, lequel en représente un mode de réalisation non limitatif.

Dans le dessin annexé:
- la figure 1 représente une vue schématique en perspective d'un système selon l'invention,
- la figure 2 représente une vue partielle schématique en perspective et en éclaté du même système, et notamment du dispositif d'implant selon l'invention,
- la figure 3 représente une vue schématique en perspective et coupe selon un plan longitudinal d'une partie du même système et du même dispositif d'implant,
- la figure 4 représente une vue schématique partielle en perspective et coupe selon un plan longitudinal du même système et du même dispositif d'implant,
- la figure 5 représente une vue partielle en perspective du même système,
- la figure 6 représente une vue en perspective d'une partie du même système,
- la figure 7 représente une vue en perspective d'une autre partie du même système,
- la figure 8 représente l'implant du même système après mise en place au niveau d'une articulation facettaire.

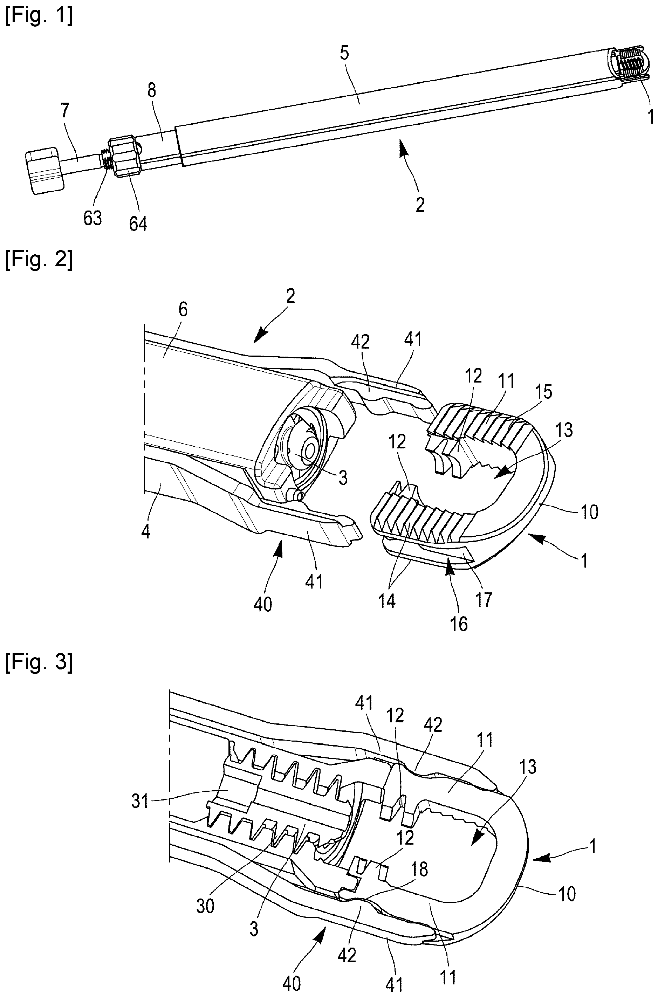

En référence à la figure 1 on peut voir un système pour la réalisation d'une arthrodèse rachidienne par voie postérieure au niveau d'une articulation facettaire selon l'invention, comprenant un implant 1 et un outil 2 de pose de cet implant 1.

En référence également aux figures 2, 3 et 4, on peut voir que l'implant 1 se présente sous la forme d'un élément plat d'épaisseur constante, de forme générale en U, comprenant donc une partie transversale 10 constituant le bord distal, de laquelle s'étendent deux branches 11. Les deux branches 11 présentent chacune du côté interne au niveau de leur extrémité libre, des éléments saillants 12 formant un taraudage pour le vissage d'une vis 3, et permettre après vissage que cette vis 3 occupe l'espace 13 séparant les deux branches 11.

On notera que de manière avantageuse, non limitativement, le bord distal 10 est aminci afin de faciliter l'insertion.

L'implant 1 comporte au niveau des deux faces extérieures 14 de chacune des deux branches 11, des reliefs 15 destinés à permettre un accrochage dans la matière osseuse, et qui se présentent sous la forme de dents.

D'autre part, la vis 3 est d'un diamètre supérieur à l'épaisseur de l'implant 1, plus particulièrement entre deux faces 14, et plus précisément, c'est uniquement le filet 30 de la vis 3, ou une partie de celui-ci, qui déborde des faces 14 de l'implant 1.

On peut également voir sur ces figures, que chacune des branches 11 présente extérieurement, c'est-à-dire du côté opposé à celui comportant les éléments saillants 12, une gorge longitudinale 16, s'étendant tout le long de la branche 11. De plus, le fond 17 de chaque gorge 16 présente un bossage concave 18.

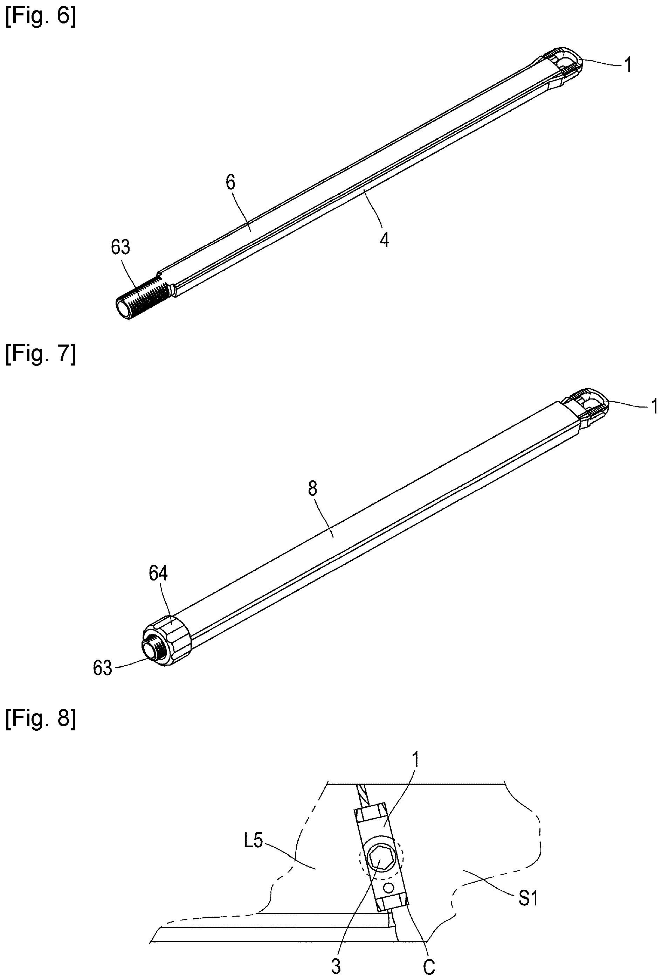

En référence maintenant à la figure 8, on peut voir un dispositif d'implant 1 selon l'invention mis en place à l'emplacement de l'articulation facettaire entre les vertèbres L5 et S1, dont le côté droit est représenté partiellement, sachant que le côté gauche, non représenté est équipé de la même manière.

Avant la mise en place, la matière osseuse des deux vertèbres est découpée, au niveau de l'articulation facettaire, en sorte de créer une cavité C, partagée entre les deux vertèbres, en l'occurrence L5 et S1, s'étendant dans le plan de contact et d'une épaisseur correspondant à celle de l'implant 1. Puis le dispositif d'implant 1 est introduit dans la cavité C.

Après introduction, le vissage de la vis 3, le filet 30, qui est de manière avantageuse auto-taraudeur, pénètre dans la matière osseuse en sorte de d'ancrer l'implant 1.

Les dimensions réduites de la cavité C, et donc également celles de l'implant peuvent rendre compliquée la mise en place de ce dernier, du fait notamment des différentes opérations à réaliser, à savoir introduction avec impaction, puis vissage. C'est dans le but de faciliter cette mise en place qu'a été conçu l'outil 2 adapté à l'implant 1, pour former le système selon l'invention.

Ainsi, l'outil 2 est composé d'une multiplicité de pièces allongées et coaxiales.

L'outil 2 comporte ainsi une première pièce 4 de forme tubulaire, dont une extrémité 40 est destinée au maintien de l'implant 1 par enserrement des branches 11. Elle comporte à cet effet deux bras parallèles 41 dimensionnés pour venir se loger chacun dans une gorge 16 de l'implant 1 au contact des fonds 17, tandis que des bossages convexes 42 viennent prendre place dans les bossages concaves 18. L'implant 1 est ainsi immobilisé entre les bras 41, à la fois transversalement par les bras 41 ainsi que par les gorges 16, et axialement au travers des bossages 42 et 18.

L'outil 2 comporte également une deuxième pièce 8 de forme tubulaire, dans laquelle peut coulisser axialement la première pièce 4, et qui en position extrême peut contraindre les branches 41 pour maintenir l'implant 1.

L'outil 2 comporte également une troisième pièce 6 de forme tubulaire, montée coulissante dans la deuxième pièce 4. Son extrémité libre 60 présente un taraudage 61 dans lequel est engagée la vis 3 lorsqu'elle est en position d'attente.

On notera que, de manière optionnelle, l'extrémité 60 de la pièce 6 comporte, faisant saillie axialement, un doigt 62, prévu apte à s'engager dans un trou borgne 19 pratiqué à l'extrémité d'une branche 11 de l'implant 1, dans un d'indexage des deux taraudages 12 et 61.

On notera également que la pièce 6 comporte à son extrémité opposée à celle comportant la vis 3, un embout fileté 63 sur lequel est vissé un écrou 64. On comprendra, au vu de la figure 6, que le serrage de l'écrou 64 sur l'embout fileté 63, a pour conséquence le déplacement du fourreau 8 sur la pièce 4, avec pour conséquence le renforcement de l'enserrement de l'implant 1 par les bras 41.

L'outil 2 comporte une quatrième pièce 7, disposée dans la pièce 6, qui est destinée à la transmission d'un couple de vissage à la vis 3, et qui présente à cet effet une extrémité 70 comportant une empreinte mâle 71 correspondant à celle femelle 31 de la vis 3, en l'occurrence de section hexagonale.

On notera que la version du système 2 tel que décrit précédemment, consiste en la version la plus aboutie, sachant qu'il est parfaitement possible que certaines des parties puissent ne pas être nécessaires selon l'intervention à réaliser.

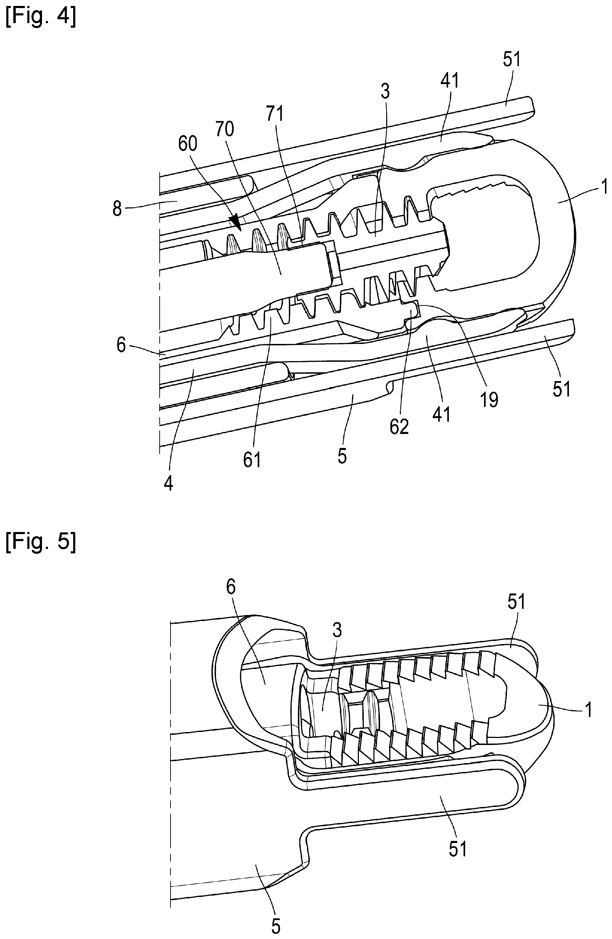

Il en est ainsi de la pièce 5, essentiellement utilisable en chirurgie percutanée, mais qui n'est pas nécessaire dans le cas d'une intervention dite « à ciel ouvert ». A cet effet elle comporte à une extrémité 50 deux extensions 51 en forme de lames qui, comme cela est visible plus précisément sur les figures 4 et 5, prennent place contre les bras parallèles 41, extérieurement à ceux-ci pour les maintenir.

## Revendications

1. Dispositif d'implant (1) pour la réalisation d'une arthrodèse rachidienne par voie postérieure au niveau d'une articulation facettaire, se présentant sous la forme d'un élément plat, d'épaisseur constante, destiné à être introduit dans une cavité (C) préalablement réalisée à l'emplacement de l'articulation facettaire à immobiliser, comprenant sur chacune de ses faces (14) des reliefs (15) formant des crans constituant des moyens d'ancrage, et associé à au moins un moyen d'ancrage complémentaire, constitué d'une vis (3) d'un diamètre supérieur à l'épaisseur dudit élément, conçue apte à être vissée dans ledit élément plat en sorte qu'au moins une partie de ses filets (30) déborde desdits faces (14), **caractérisé en ce qu'**il présente une forme de U, à savoir qu'il comporte une partie transversale (10), de laquelle s'étendent deux branches (11) séparées par un espace (13), et où ladite partie transversale (10) constitue le bord distal d'introduction dans ladite cavité (C), tandis que du côté proximal les extrémités libres desdites branches (11) comportent chacune, du côté intérieur du U, des moyens de retenue (12) aptes à permettre le vissage et l'introduction entre lesdites branches (11), de ladite vis (3) et qui font saillie de chacun des côtés internes desdites branches (11) pour s'étendre dans ledit espace (13), sans déborder l'épaisseur dudit élément plat.

2. Système pour la réalisation d'une arthrodèse rachidienne par voie postérieure au niveau d'une articulation facettaire, comportant un dispositif d'implant (1) et son outil de pose (2), dans lequel ledit implant (1) se présente sous la forme d'un élément plat, d'épaisseur constante, destiné à être introduit dans une cavité (C) préalablement réalisée à l'emplacement de l'articulation facettaire à immobiliser, comprenant sur chacune de ses faces (14) des reliefs (15) formant des crans constituant des moyens d'ancrage, et associé à au moins un moyen d'ancrage complémentaire, constitué d'une vis (3) d'un diamètre supérieur à l'épaisseur dudit élément, conçue apte à être vissée dans ledit élément plat en sorte qu'au moins une partie de ses filets (30) déborde desdits faces (14), **caractérisé en ce que** :
- ledit implant présente une forme de U, à savoir qu'il comporte une partie transversale, (10) de laquelle s'étendent deux branches (11), et où ladite partie transversale (10) constitue le bord distal d'introduction dans ladite cavité (C), tandis que du côté proximal les extrémités libres desdites branches (11) comportent chacune, du côté intérieur du U, des moyens de retenue (12) aptes à permettre le vissage et l'introduction entre lesdites branches (11), de ladite vis, (3)
- ledit outil de pose (2) comprend un ensemble de pièces coaxiales à savoir, d'une part une première pièce (4) dont une extrémité est apte à tenir ledit élément plat en le maintenant par l'extérieur de ses branches (11), d'autre part une deuxième pièce (8) dans laquelle est apte à coulisser ladite première pièce (4) jusqu'à extraction de la partie de cette dernière tenant ledit élément plat ; d'autre part encore intérieurement à ladite première pièce (4), une troisième pièce (6) dont une extrémité (60) est apte à être connectée audit élément plat et à maintenir ladite vis (3) en regard desdites branches (11) ; et d'autre part encore, intérieurement à ladite troisième pièce, une quatrième pièce (7) qui consiste en une tige dont une extrémité (70) est configurée pour engager ladite vis (3) et pouvoir l'entraîner en rotation au travers d'une action sur l'autre extrémité.

3. Système selon la revendication 2, **caractérisé en ce que** l'extrémité (40) de la première partie (4), apte à tenir l'élément plat en le maintenant par l'extérieur de ses branches (11), comporte deux bras parallèles (41), conçus aptes à enserrer lesdites branches (11) par l'extérieur.

4. Système selon la revendication 2 ou la revendication 3 **caractérisé en ce que** les branches (11) de l'élément plan présentent chacune extérieurement une gorge longitudinale (16) de guidage pour les bras (41) de la première pièce (4), et **en ce que** les fonds (17) desdites gorges (16) et l'intérieur desdits bras (41) présentent des reliefs complémentaires (18, 42) aptes à immobiliser axialement ledit élément entre lesdits bras (41) lorsque ceux-ci sont maintenus extérieurement dans la deuxième pièce (5) .

5. Système selon la revendication 4, **caractérisé en ce que** les reliefs (18, 42) consistent en des bossages.

6. Système selon l'une quelconque des revendications 2 à 5 **caractérisé en ce que** la troisième pièce (6) est de forme tubulaire tandis que son extrémité apte à maintenir la vis (30) en attente, consiste en une cavité de section transversale ronde et taraudée.

7. Système selon l'une quelconque des revendications 2 à 6 **caractérisé en ce que** l'outil (2) comporte une pièce supplémentaire (5), utilisable dans une intervention percutanée, et intégrant toutes les autres pièces. (3, 4, 6, 7, 8).

## Patentansprüche

1. Implantatvorrichtung (1) zum Durchführen einer Wirbelsäulenversteifung auf hinterem Weg auf dem Niveau eines Facettengelenks, welche die Form eines ebenen Elements mit konstanter Dicke aufweist, welches dazu vorgesehen ist, in eine Kavität (C) eingeführt zu werden, welche zuvor an der Position des zu immobilisierenden Facettengelenks angelegt wird, umfassend an jeder seiner Flächen (14) Vorsprünge (15), welche Kerben bilden, welche Verankerungsmittel darstellen, und zugeordnet zu wenigstens einem komplementären Verankerungsmittel, welches durch eine Schraube (3) mit einem Durchmesser gebildet ist, welcher größer als die Dicke des Elements ist, welche dazu ausgelegt ist, in das ebene Element derart eingeschraubt werden zu können, dass wenigstens ein Teil ihrer Gewinde (30) aus den Flächen (14) herausragt, **dadurch gekennzeichnet, dass** es eine U-Form aufweist, indem es einen transversalen Abschnitt (10) umfasst, von welchem sich zwei Zweige (11) erstrecken, welche durch einen Raum (13) getrennt sind, und wobei der transversale Abschnitt (10) den distalen Einsetzrand der Kavität (C) bildet, während an der proximalen Seite die freien Enden der Zweige (11) jeweils an der inneren Seite des U Haltemittel (12) umfassen, welche in der Lage sind, das Einschrauben und das Einführen zwischen die Zweige (11) der Schraube (3) zu erlauben, und welche von jeder der inneren Seiten der Zweige (11) abstehen, um sich in dem Raum (13) zu erstrecken, ohne über die Dicke des ebenen Elements überzustehen.

2. System zum Durchführen einer Wirbelsäulenversteifung auf hinterem Weg auf dem Niveau eines Facettengelenks, umfassend eine Implantatvorrichtung (1) und deren Anbringungswerkzeug (2), wobei das Implantat (1) in der Form eines ebenen Elements mit konstanter Dicke vorliegt, welches dazu vorgesehen ist, in eine Kavität (C) eingeführt zu werden, welche zuvor an der Position des zu immobilisierenden Facettengelenks angelegt wird, umfassend an jeder seiner Flächen (14) Vorsprünge (15), welche Kerben bilden, welche Verankerungsmittel darstellen, und zugeordnet zu wenigstens einem komplementären Verankerungsmittel, welches durch eine Schraube (3) mit einem Durchmesser gebildet ist, welcher größer als die Dicke des Elements ist, welche dazu ausgelegt ist, in das ebene Element derart eingeschraubt werden zu können, dass wenigstens ein Teil ihrer Gewinde (30) aus den Flächen (14) herausragt, **dadurch gekennzeichnet, dass**:
- das Implantat eine U-Form aufweist, indem es einen transversalen Abschnitt (10) umfasst, von welchem sich zwei Zweige (11) erstrecken, wobei der transversale Abschnitt (10) den distalen Einsetzrand der Kavität (C) bildet, während an der proximalen Seite die freien Enden der Zweige (11) jeweils an der inneren Seite des U Haltemittel (12) umfassen, welche in der Lage sind, das Einschrauben und das Einführen zwischen die Zweige (11) der Schraube (3) zu erlauben,
- das Anbringungswerkzeug (2) eine Anordnung von koaxialen Stücken umfasst, d.h. einerseits ein erstes Stück (4), von welchem ein Ende dazu in der Lage ist, das ebene Element aufzunehmen und es durch die Außenseite seiner Zweige (11) zu halten, andererseits ein zweites Stück (8), in welchem das erste Stück (4) zu gleiten in der Lage ist, bis zu einem Ausgeben des Abschnitts des letzteren, welcher das ebene Element aufnimmt; andererseits weiter innerhalb zu dem ersten Stück (4) ein drittes Stück (6), dessen eines Ende (60) in der Lage ist, mit dem ebenen Element verbunden zu sein und die Schraube (3) bezüglich der Zweige (11) zu halten, und noch andererseits innerhalb zu dem dritten Stück ein viertes Stück (7), welches aus einer Stange besteht, deren eines Ende (70) dazu eingerichtet ist, mit der Schraube (3) einzugreifen und sie in Rotation mittels einer Wirkung auf das andere Ende anzutreiben.

3. System nach Anspruch 2, **dadurch gekennzeichnet, dass** das Ende (40) des ersten Abschnitts (4), welches in der Lage ist, das ebene Element aufzunehmen und es durch die Außenseite seiner Zweige (11) zu halten, zwei parallele Arme (41) umfasst, welche dazu ausgelegt sind, dazu in der Lage zu sein, die Zweige (11) von der Außenseite zu umschließen.

4. System nach Anspruch 2 oder Anspruch 3, **dadurch gekennzeichnet, dass** die Zweige (11) des ebenen Elements jeweils außen einen longitudinalen Hals (16) zum Führen der Arme (41) des ersten Stücks (4) aufweisen, und dass die Böden (17) der Hälse (16) und die Innenseite der Arme (41) komplementäre Vorsprünge (18, 42) aufweisen, welche dazu in der Lage sind, das Element zwischen den Armen (41) axial zu immobilisieren, wenn diese extern an dem zweiten Stück (5) gehalten sind.

5. System nach Anspruch 4, **dadurch gekennzeichnet, dass** die Vorsprünge (18, 42) aus Buckeln bestehen.

6. System nach einem der Ansprüche 2 bis 5, **dadurch gekennzeichnet, dass** das dritte Stück (6) von rohrförmiger Form ist, während sein Ende, welches in der Lage ist, die Schraube (30) in Ruhe zu halten, aus einer Kavität mit rundem transversalem Querschnitt und Gewindeschnitt besteht.

7. System nach einem der Ansprüche 2 bis 6, **dadurch gekennzeichnet, dass** das Werkzeug (2) ein zusätzliches Stück (5) umfasst, welches bei einem perkutanen Eingriff verwendbar ist und alle der andern Stücke (3, 4, 6, 7, 8) integriert.

## Claims

1. An implant device (1) for performing posterior spinal arthrodesis at a facet joint, in the form of a flat element, of constant thickness, intended to be inserted into a cavity (C) previously produced at the location of the facet joint to be immobilised, comprising, on each of its faces (14), reliefs (15) forming notches constituting anchoring means, and which is associated with at least one complementary anchoring means composed of a screw (3) with a diameter greater than the thickness of said element, designed to be able to be screwed into said flat element such that at least some of its threads (30) protrude from said faces (14), **characterised in that** it has a U-shape, namely it comprises a transverse part (10) from which two branches (11), separated by a gap (13), extend, and where said transverse part (10) constitutes the distal insertion edge into said cavity (C), while on the proximal side, the free ends of said branches (11) each comprise, on the inner side of the U, retention means (12) that are able to permit the screwing and insertion of said screw (3) between said branches (11), and which project from each of the inner sides of said branches (11) to extend into said gap (13), without exceeding the thickness of said flat element.

2. A system for performing posterior spinal arthrodesis at a facet joint, comprising an implant device (1) and its positioning tool (2), wherein said implant (1) is in the form of a flat element, of constant thickness, intended to be inserted into a cavity (C) previously produced at the location of the facet joint to be immobilised, comprising, on each of its faces (14), reliefs (15) forming notches constituting anchoring means, and associated with at least one complementary anchoring means composed of a screw (3) with a diameter greater than the thickness of said element, designed to be able to be screwed into said flat element such that at least some of its threads (30) protrude from said faces (14), **characterised in that**:
- said implant has a U-shape, namely it comprises a transverse part (10), from which two branches (11) extend, and where said transverse part (10) constitutes the distal insertion edge into said cavity (C), while on the proximal side, the free ends of said branches (11) each comprise, on the inner side of the U, retention means (12) that are able to permit the screwing and insertion of said screw (3) between said branches (11),
- said positioning tool (2) comprises a set of coaxial pieces, namely, firstly, a first piece (4) of which one end is able to hold said flat element by holding it from the outside of its branches (11), secondly, a second piece (8) wherein said first piece (4) is able to slide until the part thereof holding said flat element is extracted; thirdly, inside said first piece (4), a third piece (6) of which one end (60) is able to be connected to said flat element and to hold said screw (3) facing said branches (11); and, fourthly, inside said third piece, a fourth piece (7) which consists of a rod of which one end (70) is configured to engage said screw (3) and to be able to rotate it via an action on the other end.

3. The system according to claim 2, **characterised in that** the end (40) of the first piece (4) that is able to hold the flat element by holding it from the outside of its branches (11) comprises two parallel arms (41), designed to be able to grip said branches (11) from the outside.

4. The system according to claim 2 or claim 3, **characterised in that** the branches (11) of the planar element each have externally a longitudinal groove (16) for guiding the arms (41) of the first piece (4), and **in that** the bottoms (17) of said grooves (16) and the inside of said arms (41) have complementary reliefs (18, 42) that are able to immobilise said element axially between said arms (41) when the latter are held externally in the second piece (5).

5. The system according to claim 4, **characterised in that** the reliefs (18, 42) consist of bosses.

6. The system according to any one of claims 2 to 5, **characterised in that** the third piece (6) has a tubular shape, while its end that is able to hold the screw (30) in a holding position, consists of a cavity with a round and tapped cross-section.

7. The system according to any one of claims 2 to 6, **characterised in that** the tool (2) comprises an additional piece (5) that can be used in a percutaneous procedure, and incorporating all the other pieces. (3, 4, 6, 7, 8).
